# FASCICULE DE BREVET EUROPEEN

(11) **EP 3 060 629 B1**
(45) Date de publication et mention de la délivrance du brevet: **30.01.2019**
(21) Numéro de dépôt: 14793481.4
(22) Date de dépôt: 23.10.2014
(51) Int. Cl.: F25J 3/02, C10L 3/00, C07C 7/00, C07C 5/327, C10G 9/00, C10G 70/04, C10G 5/06, C07C 7/09

(54) **PROCÉDÉ DE FRACTIONNEMENT D'UN COURANT DE GAZ CRAQUÉ, METTANT EN OEUVRE UN COURANT DE RECYCLE INTERMÉDIAIRE, ET INSTALLATION ASSOCIÉE**
VERFAHREN ZUR FRAKTIONIERUNG EINES STROMES VON SPALTGAS UNTER VERWENDUNG EINES ZWISCHENSRÜCKFÜHRUNGSSTROMES UND ZUGEHÖRIGE ANLAGE
METHOD FOR FRACTIONATING A STREAM OF CRACKED GAS, USING AN INTERMEDIATE RECIRCULATION STREAM, AND RELATED PLANT

(30) Priorité: 23.10.2013 FR 1360349
(43) Date de publication de la demande: 31.08.2016
(73) Titulaire: Technip France, 92400 Courbevoie (FR)
(72) Inventeur: DESTOUR, Bruno, 92500 Rueil Malmaison (FR); SIMON, Yvon, 78570 Andresy (FR); DADOU, Aurélia, 92800 Puteaux (FR); CHAZALLET, David, 76000 Rouen (FR)
(74) Mandataire: Lavoix
(86) Numéro de dépôt international: PCT/EP2014/072767
(87) Numéro de publication internationale: WO 2015/059233

(56) Documents cités:
- WO-A2-2011/051614
- US-A1- 2006 004 242
- US-A1- 2007 199 865
- US-B1- 6 271 433

## Description

La présente invention concerne un procédé de fractionnement d'un courant de gaz craqué issu d'une installation de pyrolyse d'hydrocarbures pour obtenir une coupe riche en éthylène et un courant de combustible pauvre en hydrocarbures en C₂⁺ selon le préambule de la revendication 1.

Le gaz craqué est issu d'une installation de pyrolyse d'hydrocarbures tel qu'un four de vapocraquage. Le gaz introduit dans l'installation de pyrolyse présente avantageusement entre 60 % et 70% d'éthane, en association avec du propane, du butane, du naphta, du et/ou du gasoil.

Le procédé du type précité est destiné à traiter le gaz craqué pour obtenir une coupe d'éthylène présentant une teneur en éthylène supérieure à 99,95 % en moles, en récupérant plus de 99,5 % en moles de l'éthylène contenu dans le gaz craqué.

Un procédé du type précité qui permet d'obtenir de telles performances est décrit par exemple dans EP 1 215 459.

Ce procédé est destiné à être mis en oeuvre pour traiter de très grands volumes de gaz craqué, par exemple supérieur à 50 tonnes, notamment supérieur à 100 tonnes par heure.

Pour garantir à la fois une très grande pureté du courant d'éthylène produit et un taux de récupération d'éthylène maximal, il est nécessaire de refroidir le gaz traité jusqu'à des températures inférieures à - 100°C et notamment inférieures à - 120°C.

A cet effet, le courant de gaz craqué est mis en relation d'échange thermique avec de l'éthylène circulant dans un cycle de réfrigération externe.

Le cycle de réfrigération à l'éthylène comprend généralement trois niveaux thermiques, avec un premier échangeur thermique à environ - 50°C, un deuxième échangeur thermique à environ -75°C et un troisième échangeur thermique à environ - 100°C.

Après chaque échange thermique, le gaz craqué partiellement condensé est introduit dans un séparateur pour évacuer le liquide formé.

Les liquides recueillis, qui sont généralement riches en hydrocarbures en C₂⁺, sont envoyés vers une unité de traitement comportant au moins une colonne de fractionnement. La colonne de fractionnement produit le courant contenant l'éthylène récupéré par le procédé cryogénique.

L'utilisation d'un cycle de refroidissement externe à base d'éthylène, à trois niveaux thermiques augmente significativement la consommation énergétique du procédé. L'investissement nécessaire à l'installation du cycle est en outre significatif.

Pour pallier ce problème, WO2011/051614 décrit un procédé, dans lequel le troisième niveau froid du cycle de réfrigération à base d'éthylène est supprimé et est remplacé par une double détente du courant de combustible dans deux appareils successifs de détente dynamique pour fournir les frigories nécessaires au refroidissement à basse température du courant de gaz craqué.

Ce procédé supprime donc un niveau thermique du cycle de réfrigération à l'éthylène, ce qui limite l'investissement nécessaire à sa mise en oeuvre. Il conserve néanmoins toute son efficacité en termes de taux de récupération d'éthylène, tout en présentant des performances énergétiques améliorées.

Dans certains cas, il est souhaitable de réduire encore l'investissement nécessaire. Ceci est le cas par exemple pour des petites unités de production d'éthylène, dans lesquelles l'installation et la mise en fonctionnement d'un cycle à l'éthylène est particulièrement onéreuse, puisqu'elle peut représenter jusqu'à 5 % du prix des équipements de l'unité.

Par ailleurs, la fourniture initiale d'éthylène pour démarrer l'unité, et sa manipulation lors de la mise en service du cycle de réfrigération peut s'avérer compliquée, notamment lorsqu'il est difficile d'acheminer de l'éthylène jusqu'à l'unité.

Un but de l'invention est donc d'obtenir un procédé de fractionnement de gaz craqué qui nécessite un investissement encore inférieur pour s'adapter notamment aux petites unités, tout en offrant un taux de récupération en éthylène très élevé et des performances énergétiques satisfaisantes.

À cet effet, l'invention a pour objet un procédé selon la revendication 1.

Le procédé selon l'invention peut comprendre l'une ou plusieurs des caractéristiques des revendications 2 à 13 ou l'une des caractéristiques suivantes, prise(s) isolément ou suivant toute combinaison techniquement possible :
- le procédé comprend les étapes suivantes :
   * passage du courant de combustible partiellement détendu issu de l'échangeur intermédiaire dans un deuxième appareil de détente dynamique pour former un courant de combustible détendu ;
   * réchauffage du courant de combustible détendu issu du deuxième appareil de détente dynamique dans l'échangeur thermique aval et dans l'échangeur thermique intermédiaire et dans l'échangeur thermique amont ;
   * compression du courant de combustible détendu réchauffé dans au moins un compresseur accouplé à au moins une turbine de détente du premier appareil de détente dynamique ou/et du deuxième appareil de détente dynamique pour former le courant de combustible pauvre en hydrocarbures en C₂⁺;
- la puissance thermique nécessaire au refroidissement du courant amont de gaz craqué vers la première température est fournie dans l'échangeur thermique amont par échange thermique avec le courant de recycle intermédiaire et avantageusement par échange thermique avec le courant de combustible détendu, sans échange thermique avec un fluide réfrigérant externe circulant dans un cycle de réfrigération ;
- la puissance thermique nécessaire au refroidissement du courant intermédiaire de gaz craqué vers la deuxième température est fournie dans l'échangeur thermique intermédiaire par échange thermique avec le courant de combustible haute pression, par échange thermique avec le courant de combustible partiellement détendu, par échange thermique avec le courant de recycle intermédiaire, et avantageusement par échange thermique avec le courant de combustible détendu, sans échange thermique avec un fluide réfrigérant externe circulant dans un cycle de réfrigération ;
- la puissance thermique nécessaire au refroidissement du courant aval de gaz craqué jusqu'à la troisième température est fournie dans l'échangeur thermique aval par échange thermique avec le courant de combustible à haute pression, par échange thermique avec le courant de combustible partiellement détendu, avantageusement par échange thermique avec le courant de combustible détendu, et avantageusement par échange thermique avec le courant gazeux de recyclage, sans échange thermique avec un fluide réfrigérant externe circulant dans un cycle de réfrigération ;
- la totalité du courant de combustible haute pression réchauffé issu de l'échangeur intermédiaire est introduite dans le premier appareil de détente dynamique, la totalité du courant de combustible partiellement détendu réchauffé issu de l'échangeur intermédiaire étant introduite dans le deuxième appareil de détente dynamique ;
- l'étape de traitement comprend l'introduction d'au moins un courant formé à partir du liquide amont, du liquide intermédiaire ou/et du liquide aval dans une colonne de fractionnement et la production dans la colonne de fractionnement d'un courant riche en éthylène destiné à former la coupe riche en éthylène ;
- le courant de tête issu de la colonne de fractionnement est convoyé en totalité vers l'échangeur thermique amont et avantageusement vers un échangeur amont de réchauffage, avant d'être mélangé au gaz craqué brut, sans qu'une fraction de ce courant ne soit condensée pour être envoyée en reflux dans la colonne de fractionnement ;
- la teneur molaire en hydrogène dans le courant de combustible haute pression est supérieure à 75% ;
- la première température est inférieure à - 63° C, la deuxième température est inférieure à - 85°C, et la troisième température est inférieure à - 125°C ;
- le procédé comporte une étape de formation du courant de combustible pauvre en hydrocarbures en C₂⁺ à partir du courant de combustible partiellement détendu par compression dans au moins un compresseur ;
- le procédé comporte une étape de détente du courant de combustible partiellement détendu, de réchauffage dans au moins un échangeur thermique.

L'invention a également pour objet une installation de fractionnement d'un courant de gaz craqué issu d'une installation de pyrolyse d'hydrocarbures pour obtenir une coupe riche en éthylène et un courant de combustible pauvre en hydrocarbures en C₂⁺, selon la revendication 14.

L'invention sera mieux comprise à la lecture de la description qui va suivre, donnée uniquement à titre d'exemple, et faite en se référant aux dessins annexés, sur lesquels :
- la figure 1 est un schéma synoptique fonctionnel d'une première installation de fractionnement selon l'invention, destinée à la mise en oeuvre d'un premier procédé selon l'invention, et
- la figure 2 est un détail d'une variante d'installation de fractionnement selon l'invention.

Dans tout ce qui suit, une même référence désigne un courant circulant dans une conduite et la conduite qui transporte ce courant. Par ailleurs, sauf indication contraire, les pourcentages sont des pourcentages molaires et les pressions s'entendent en bars relatifs.

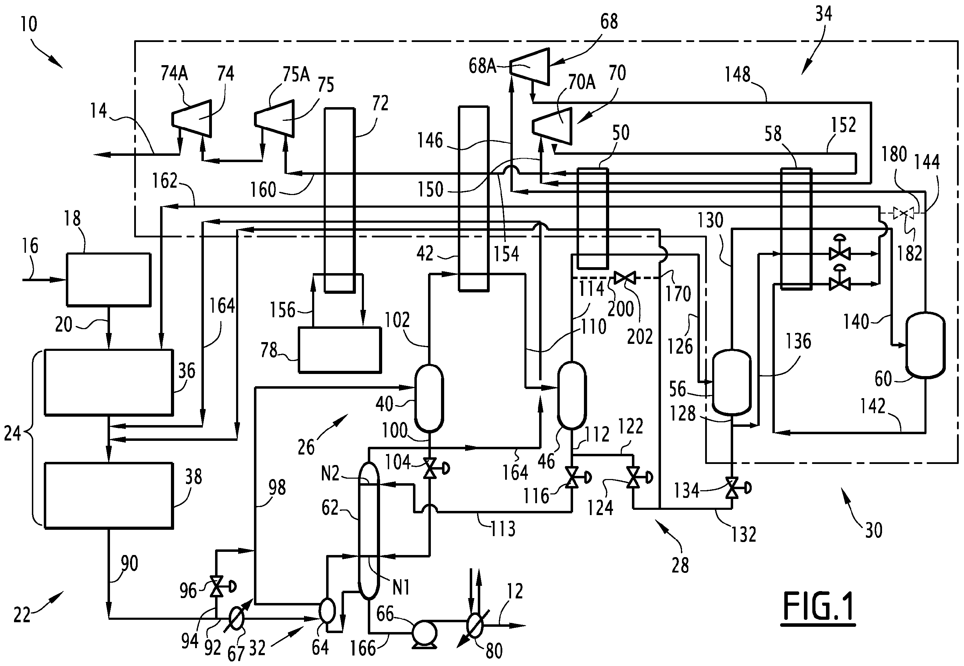

Une première unité 10 de vapocraquage selon l'invention est représentée sur la figure 1.

Cette unité 10 est destinée à former une coupe 12 riche en éthylène et un courant 14 de gaz combustible pauvre en hydrocarbures en C₂⁺, à partir d'une charge 16.

L'unité 10 comprend une installation 18 de pyrolyse d'hydrocarbures comportant un four de vapocraquage destiné à produire un courant 20 de gaz craqué brut. Elle comporte en outre une installation 22 de fractionnement du gaz traité brut pour former le courant de gaz de combustible 14 et la coupe riche en éthylène 12.

La charge 16 est avantageusement formée d'au moins 60% en moles d'éthane, en association avec du propane, du butane, du naphta et/ou du gasoil.

Le four de vapocraquage 18 est propre à faire circuler la charge 16 pour la chauffer à une température supérieure à 800°C. Ceci provoque le craquage thermique des molécules d'hydrocarbures contenues dans la charge 16 afin de former le courant de gaz craqué brut 20.

L'installation de fractionnement 22 comporte successivement un étage 24 de refroidissement et de compression, et un ensemble amont 26, un ensemble intermédiaire 28 et un ensemble aval 30 de refroidissement et de séparation du gaz craqué.

Selon l'invention, l'ensemble amont 26, l'ensemble intermédiaire 28, et l'ensemble aval 30 sont dépourvus de cycle de refroidissement externe, notamment à l'éthylène.

L'installation 22 comporte en outre un ensemble 32 de traitement des liquides formés dans les ensembles 26 à 30, et un ensemble 34 de détente et de réchauffage du gaz combustible.

L'étage de compression et de refroidissement 24 comporte un dispositif de refroidissement (non représenté), un compresseur primaire 36 et un compresseur secondaire 38, le compresseur secondaire 38 étant disposé en aval du compresseur primaire 36.

L'ensemble amont 26 de refroidissement et de séparation comporte un premier ballon séparateur amont 40, un échangeur thermique amont 42, et un deuxième ballon séparateur amont 46.

L'ensemble intermédiaire 28 de refroidissement et de séparation comporte, d'amont en aval, un échangeur thermique intermédiaire 50, et un ballon séparateur intermédiaire 56. Dans cet exemple, l'ensemble intermédiaire 28 comprend un échangeur thermique intermédiaire 50 unique.

L'ensemble aval 30 de refroidissement et de séparation comprend un échangeur thermique aval 58, et un ballon séparateur aval 60 destiné à produire le courant de gaz combustible.

L'ensemble 32 de traitement des liquides comporte une colonne de fractionnement 62, un échangeur thermique de rebouillage 64, et une pompe 66 de fond de colonne.

Dans cet exemple, l'ensemble 32 comprend en outre avantageusement un échangeur thermique d'appoint 67, propre à fournir une puissance thermique de refroidissement variable en fonction de la puissance thermique de refroidissement fournie par l'échangeur thermique de rebouillage 64. Cet échangeur thermique d'appoint 67 est par exemple alimenté en fluide réfrigérant par du propylène.

L'ensemble 34 de détente et de réchauffage comprend un premier appareil de détente dynamique 68, et avantageusement, un deuxième appareil de détente dynamique 70. Les appareils 68, 70 présentent chacun au moins une turbine de détente dynamique 68A, 70A.

L'ensemble 34 de détente et de réchauffage comporte en outre un échangeur thermique 72 de réchauffage, un premier appareil 74 de compression et un deuxième appareil 75 de compression, les appareils 74 et 75 présentant chacun au moins un compresseur 74A et 75A, qui sont chacun accouplés à une turbine de détente 68A, 70A respective du premier appareil de détente dynamique 68 et du deuxième appareil de détente dynamique 70.

L'échangeur thermique de réchauffage 72 refroidit un fluide réfrigérant circulant dans un cycle 78 de réfrigération au propylène. Le cycle au propylène 78 comporte un échangeur thermique de pied 80 placé en aval de la pompe 66 de fond de colonne. L'échangeur 80 peut être intégré dans l'échangeur 72.

Un premier procédé selon l'invention, mis en oeuvre dans l'unité 10 pour traiter le courant de gaz craqué issu du vapocraquage d'une charge 16, va maintenant être décrit.

Initialement, la charge 16 contenant majoritairement de l'éthane est introduite dans le four de vapocraquage 18 pour être chauffée à une température supérieure à 800°C et subir un craquage thermique.

Un courant de gaz craqué brut 20 est extrait du four 18 à une température supérieure à 800°C et à une pression supérieure à 1 bars.

Ce courant 20 est ensuite refroidi et introduit dans le compresseur primaire 36 pour être comprimé à une pression supérieure à 10 bars sensiblement inférieure à la pression dans la colonne de fractionnement 62, puis dans le compresseur secondaire 38 pour être comprimé à une pression supérieure à 30 bars.

Le courant de gaz craqué 90 comprimé issu du compresseur secondaire 38 est ensuite séparé en une première fraction de rebouillage 92 et en une deuxième fraction 94.

Dans l'exemple représenté sur la figure 1, la fraction de rebouillage 92 est tout d'abord introduite dans l'échangeur thermique d'appoint 67 pour y être partiellement refroidie, par une puissance thermique commandée en fonction de la puissance thermique requise par l'échangeur thermique de rebouillage 64.

La fraction de rebouillage 92 est introduite dans l'échangeur thermique 64 de rebouillage pour y être refroidie et partiellement condensée.

La deuxième fraction 94 est passée à travers une première vanne 96 de contrôle de débit, avant d'être mélangée avec la fraction 92 de rebouillage issue de l'échangeur 64 pour former un courant de gaz craqué comprimé 98 partiellement condensé.

Dans une variante du procédé, le courant de gaz craqué 90 peut avantageusement circuler, partiellement ou en totalité, au travers de l'échangeur thermique de réchauffage 72 et/ou de l'échangeur thermique d'appoint 67, avant la séparation en les fractions 92 et 94, afin de se refroidir dans l'échangeur 72 et/ou dans l'échangeur thermique d'appoint 67.

Le rapport molaire de la première fraction de rebouillage 92 à la deuxième fraction 94 est compris entre 0% et 20%. Le courant de gaz craqué 98 partiellement condensé contient au moins 15% molaire de liquide. Il présente une température inférieure à -30 °C.

Puis, le courant 98 est introduit dans le premier ballon séparateur amont 40 pour former un premier liquide amont 100 et un courant amont de gaz craqué 102.

Le premier liquide amont 100 est prélevé dans le fond du premier ballon séparateur 40 et est introduit à un niveau inférieur N1 de la colonne de fractionnement 62, après passage et détente dans une deuxième vanne 104 de contrôle de débit.

La pression dans la colonne de fractionnement 62 est avantageusement comprise entre 10 bars et 14 bars.

Le courant amont de gaz craqué 102 est ensuite introduit dans l'échangeur thermique amont 42. Il est refroidi et partiellement condensé dans l'échangeur thermique amont 42.

Selon l'invention, et comme on le verra plus bas, le refroidissement et la condensation partielle du courant amont de gaz craqué 102 ne nécessite pas l'utilisation d'un fluide réfrigérant externe, circulant dans un cycle de réfrigération, notamment à l'éthylène.

Le courant amont de gaz craqué 102 est refroidi jusqu'à une première température inférieure à - 63°C et notamment sensiblement comprise entre - 63°C et - 78°C dans l'échangeur thermique amont 42.

À la sortie de l'échangeur thermique amont 42, un courant amont 110 partiellement condensé de gaz craqué est obtenu. Ce courant amont de gaz craqué partiellement condensé 110 est introduit dans le deuxième ballon séparateur amont 46.

La teneur molaire en liquide dans le courant amont de gaz craqué partiellement condensé 110 est comprise entre 30% et 60%.

Dans le deuxième ballon séparateur amont 46, le courant amont de gaz craqué partiellement condensé 110 se sépare en un deuxième liquide amont 112 et en un courant gazeux intermédiaire 114 de gaz craqué refroidi à une première température inférieure à -63°C.

Le deuxième liquide amont 112 est récupéré au fond du deuxième ballon séparateur amont 46. Une première fraction 113 du deuxième liquide amont 112 passe dans une troisième vanne 116 de contrôle de débit et est introduite, après détente, à un niveau N2 de la colonne de fractionnement 62 situé au-dessus du niveau N1, avantageusement en tête de la colonne 62.

Le courant intermédiaire de gaz craqué 114 est introduit dans le premier échangeur thermique intermédiaire 50 pour y être refroidi à une température inférieure à - 85°C et former un courant intermédiaire 126 partielement condensé de gaz craqué. Le courant 126 présente une température inférieure à - 85°C, notamment comprise entre - 105 °C est -120 °C et une teneur en liquide compris entre 5% en moles et 30% en moles.

Dans le procédé selon l'invention, le refroidissement du courant 114 s'effectue par simple passage dans un échangeur thermique intermédiaire 50, sans avoir à passer dans d'autre échangeur thermique intermédiaire, ou sans contact thermique avec un fluide externe de réfrigération circulant dans un cycle de réfrigération externe, notamment un cycle à l'éthylène.

Le courant intermédiaire partiellement condensé de gaz craqué 126 est ensuite introduit dans le ballon séparateur intermédiaire 56 pour former un liquide intermédiaire 128 et un courant gazeux aval 130 de gaz craqué.

Selon l'invention, au moins un courant de recycle intermédiaire détendu 170 est formé à partir d'un liquide 112, 128 obtenu lors des étapes de refroidissement amont, ou/et de refroidissement intermédiaire, en amont de l'étape de refroidissement aval.

Dans cet exemple, le courant de recycle intermédiaire détendu 170 est formé à partir d'une deuxième fraction 122 du liquide amont 112 et à partir d'une première fraction 132 du liquide intermédiaire 128.

À cet effet, la deuxième fraction 122 du liquide amont 112 est introduite et détendue dans une quatrième vanne 124 de contrôle. Le débit molaire de la deuxième fraction 122 du liquide amont 112 représente avantageusement entre 50 % molaire et 90% molaire du débit molaire du liquide amont 112.

La première fraction 132 du deuxième liquide intermédiaire 128 est introduite et détendue dans une cinquième vanne 134 de contrôle de débit.

Une deuxième fraction 136 de recirculation du deuxième liquide intermédiaire 128 est sous-refroidie dans l'échangeur thermique aval 58, comme on le verra plus bas.

Le débit molaire de la première fraction 132 du liquide intermédiaire 128 représente avantageusement entre 70 % molaire et 100 % molaire du débit molaire du liquide intermédiaire 128.

Les fractions 122, 132 sont ensuite mélangées pour former le courant de recycle intermédiaire 170.

La température du courant de recycle intermédiaire 170 est comprise entre -75 °C et -95 °C, après détente dans les vannes 124, 134, et avant introduction dans un échangeur thermique 50.

Le courant de recycle intermédiaire 170 est ensuite réchauffé à travers l'échangeur thermique intermédiaire 50, l'échangeur thermique amont 42 et l'échangeur thermique de réchauffage 72.

Il est ensuite réinjecté dans le gaz craqué brut 20, à l'étage de compression et de refroidissement 24, en amont d'au moins un compresseur 36, 38.

Selon l'invention, le courant de recycle intermédiaire 170 est réinjecté dans le gaz craqué brut 20 entre le premier compresseur 36 et le deuxième compresseur 38.

Selon l'invention, le courant de recycle intermédiaire 170 n'est pas totalement détendu. Le courant 170 est en effet détendu à moyenne pression permettant de le réinjecter après le premier compresseur 36.

Ainsi, la pression du courant de recycle intermédiaire détendu 170, après détente dans les vannes 124, 134 et avant son passage dans l'échangeur thermique intermédiaire 50 est supérieure à 15 % et est avantageusement comprise entre 20 % et 50 % de la pression du courant de gaz craqué comprimé 90.

Avantageusement, la pression du courant de recycle intermédiaire détendu 170 est supérieure à 5 bars, et est notamment comprise entre 5 bars et 30 bars, notamment entre 8 bars et 15 bars.

Le débit molaire du courant de recycle intermédiaire 170 est significatif. Ce débit molaire est supérieur à 25 % du débit molaire du courant de gaz craqué brut 20 avant passage dans l'étage de refroidissement et de compression 24. Ce débit molaire est notamment compris entre 30 % et 60 % du débit molaire du courant de gaz craqué brut 20, avantageusement entre 40 % et 60 % du débit molaire du courant de gaz craqué brut 20.

Le courant de recycle intermédiaire 170 est riche en éthylène. La teneur molaire en éthylène dans le courant de recycle intermédiaire 170 est avantageusement supérieure à 50 % et est notamment comprise entre 55 % et 65 %.

Typiquement, la teneur molaire en éthane dans le courant de recycle intermédiaire 170 est comprise entre 15 % et 30 %, la teneur molaire en méthane dans le courant de recycle intermédiaire 170 étant comprise entre 10 % et 20 %.

Avantageusement, le courant de recycle intermédiaire 170 comporte moins de 3 % molaire d'hydrogène, et moins de 1 % molaire de composés comportant trois ou plus molécules de carbone.

Un avantage majeur de cette invention est que la composition molaire typique du courant 170 est sensiblement constante au cours du temps, quelle que soit la teneur d'éthane dans le courant de gaz craqué brut 20. Cette composition varie peu avec la conversion de la charge 16 dans le four 18. Le procédé de fractionnement selon l'invention est donc stabilisé par l'ajout du courant 170 dans le courant de gaz craqué brut 20, même si la composition du gaz craqué brut 20 varie.

Le courant 170 enrichit le courant de gaz craqué brut 20 en éthylène, après sa réinjection dans le courant 20.

Ainsi, le rapport de la teneur molaire en éthylène à la teneur molaire en hydrogène dans le courant de gaz craqué brut comprimé 90, après réintroduction du courant de recycle intermédiaire détendu 170 est supérieur à 1,3 fois, notamment supérieure à 1,5 fois, le rapport de la teneur molaire en éthylène à la teneur molaire en hydrogène dans le courant de gaz craqué brut 20, avant réinjection du courant de recycle intermédiaire détendu 170.

Ceci présente l'effet bénéfique d'augmenter la pression partielle en éthylène et de condenser l'éthylène dans les courants 90, 98, 102, 114, 130 à une température plus élevée, réduisant le coût énergétique.

Le courant aval de gaz craqué 130 est introduit dans l'échangeur thermique aval 58 pour y être refroidi et former un courant aval 140 de gaz craqué partiellement condensé. La température du courant 140, à la sortie de l'échangeur thermique aval 58 est inférieure à -125°C et est notamment comprise entre - 125°C et -140°C.

Le courant 140 est ensuite introduit dans le ballon séparateur aval 60 pour y être séparé en un liquide aval 142 et en un courant de gaz combustible 144 à haute pression destiné à être détendu. Le courant de gaz de combustible 144 comporte plus de 75% en moles d'hydrogène et moins de 0.5% en moles d'hydrocarbures en C₂⁺.

Le courant 144 est introduit une première fois dans l'échangeur thermique aval 58 pour se réchauffer par échange thermique à contre-courant avec le courant aval 130 de gaz craqué refroidi, puis dans l'échangeur thermique 50 pour y être réchauffé par échange thermique avec le premier courant intermédiaire de gaz craqué 114 jusqu'à une température supérieure à - 85°C.

Le courant de gaz combustible à haute pression 146 réchauffé à une température supérieure à -85°C est ensuite introduit dans une turbine de détente dynamique 68A du premier appareil de détente dynamique 68 pour être détendu jusqu'à une pression inférieure à 12 bars et former un courant 148 de gaz combustible à pression intermédiaire.

La température du courant 148 est inférieure à -115°C. Le courant 148 est alors introduit à nouveau dans l'échangeur thermique aval 58, puis dans l'échangeur thermique intermédiaire 50 pour se réchauffer successivement par échange thermique respectivement avec le courant 130, et le courant 114, comme décrit précédemment.

Ce passage du courant 148 à travers les échangeurs 50, 58 s'effectue entre une turbine 68A du premier appareil 68 et une turbine 70A du deuxième appareil 70.

Avantageusement, le courant 150 de gaz combustible réchauffé à pression intermédiaire est ensuite introduit dans une turbine 70A de détente dynamique du deuxième appareil 70 de détente dynamique pour y être détendu à une pression inférieure à 4 bars et former un courant de gaz combustible 152 à basse pression refroidi.

La température du courant 152 est alors inférieure à -115°C, et sa pression est inférieure à 4 bars.

Le courant 152 est ensuite introduit successivement dans l'échangeur thermique aval 58, puis dans l'échangeur thermique 50 pour y être réchauffé à contre-courant respectivement du courant 130, et du courant 114 comme décrit plus haut.

Le courant de gaz combustible 154 basse pression réchauffé issu du premier échangeur thermique intermédiaire 50 est ensuite introduit successivement dans l'échangeur thermique amont 42 pour être placé en relation d'échange thermique avec le courant gazeux de gaz craqué 102, puis dans l'échangeur thermique de réchauffage 72.

Dans l'échangeur thermique de réchauffage 72, le courant 154 se réchauffe par échange thermique avec le fluide réfrigérant 156 au propylène circulant dans le cycle de réfrigération 78.

Le courant 160 de gaz combustible réchauffé à basse pression issu de l'échangeur 72 présente ainsi une pression proche de la pression atmosphérique.

Le courant 160 est ensuite introduit successivement dans le compresseur 75A du deuxième appareil de compression 75, puis dans le compresseur 74A de l'appareil de compression aval 74 pour former le courant de combustible 14 destiné à alimenter le réseau de l'installation. La pression du courant 14 est supérieure à 5 bars.

La teneur en éthylène dans le gaz combustible 144 haute pression, comme dans le gaz combustible 14 est inférieure à 0,5% molaires. Le taux de récupération d'éthylène dans l'installation est supérieur à 99,5%.

Le courant de combustible 14 comprend avantageusement plus de 99% du méthane contenu dans le courant de gaz craqué brut 20.

Le liquide aval 142 comporte plus de 25% en moles d'hydrocarbures en C₂⁺. Il est introduit dans l'échangeur thermique aval 58 pour y être sous-refroidi jusqu'à une température inférieure à -130°C.

Après leur passage dans l'échangeur 58, les liquides 136, 142 sont détendus, puis sont mélangés et sont introduits successivement dans les échangeurs thermiques 58, 50, 42 et 72 pour se réchauffer et s'évaporer par échange thermique avec les courants respectifs circulant dans ces échangeurs.

Ils forment alors un courant gazeux 162 de recyclage réchauffé qui présente une température supérieure à 10°C. Le courant gazeux 162 est réintroduit dans le courant de gaz craqué brut 20, dans le compresseur primaire 36.

Le courant gazeux de recyclage 162 est détendu à basse pression. Il est ainsi détendu à une pression inférieure à celle du courant de recycle intermédiaire 170, notamment inférieure à 40 % de la pression du courant de recycle intermédiaire 170.

La pression du courant gazeux de recyclage 162, avant passage dans l'échangeur thermique aval 58, est inférieure à 5 bars, notamment inférieure à 2 bars.

La colonne de fractionnement 62 produit un courant de tête 164 riche en méthane et un courant de pied 166 riche en éthylène.

Le courant de tête 164 est introduit, après réchauffage dans l'échangeur thermique amont 42, puis après réchauffage dans l'échangeur thermique de réchauffage 72, dans le courant de gaz craqué brut 20, entre le compresseur primaire 36 et le compresseur secondaire 38.

Le courant de pied 166 issu de la colonne de fractionnement 62 est pompé par la pompe 66, avant d'être introduit dans l'échangeur thermique de récupération 80 (qui peut être intégré dans l'échangeur 72). Il est alors réchauffé au contact du propylène formant le fluide réfrigérant du cycle 78. Après passage dans l'échangeur 80, la coupe 12 riche en éthylène est formée. Cette coupe 12 comporte plus de 99,5% en moles de l'éthylène contenu dans le courant de gaz craqué brut 20.

Selon l'invention, le courant amont de gaz craqué 102, qui a été refroidi à une température comprise entre -30°C et -50°C grâce à la réfrigération fournie par les échangeurs 64 et 67, est ensuite refroidi jusqu'à une température inférieure à - 63°C dans l'échangeur 42, puis jusqu'à une température inférieure à - 85°C dans l'échangeur 50, et ensuite jusqu'à une température inférieure à - 125°C dans l'échangeur 58.

Ce refroidissement s'effectue exclusivement par échange thermique avec le courant de gaz combustible 144 à haute pression, avec le courant de gaz combustible partiellement détendu 148, avantageusement avec le courant de gaz combustible détendu 152, et par le réchauffage du courant de recyclage 162, associé au réchauffage du courant de recycle intermédiaire 170.

Il n'est donc pas nécessaire de prévoir un cycle de réfrigération externe, notamment à l'éthylène. Ceci diminue la consommation énergétique du procédé et l'investissement nécessaire pour sa mise en oeuvre.

Le procédé selon l'invention est donc particulièrement adapté à des unités de faible et moyenne capacité pour lesquelles les coûts d'investissement et d'entretien doivent être réduits au minimum.

Il est ainsi possible de disposer d'une puissance spécifique de réfrigération faible, et ce en conservant un taux de récupération d'éthane supérieur à 99,5 % et en produisant une coupe 12 riche en éthylène.

Ce résultat est obtenu en diminuant l'investissement nécessaire pour l'installation, puisqu'il n'est plus nécessaire de prévoir des équipements nécessaires à la mise en oeuvre d'un cycle de réfrigération externe à l'éthylène, comme des compresseurs, des échangeurs et des ballons séparateurs.

Le procédé selon l'invention peut également être démarré sans qu'il soit nécessaire de fournir initialement de l'éthylène, par exemple dans des lieux difficilement accessibles.

Des exemples de température, de pression, de débit molaire et de composition des flux 20, 90, 170 et 144 sont donnés à titre illustratif dans le tableau ci-dessous.

| | | | | |
|---|---|---|---|---|
| Flux | 20 | 90 | 170 | 144 |
| Débit (base 100) | 100 | 170 | 50 | 2 |
| Température (°C) | Ambiante | Ambiante à - 20°C | -75°C à -95°C | -130°C à -155°C |
| Pression (bar) | 0,8 | 35 | 10,5 | 1 |
| Composition | | | | |
| Hydrogène | 36% | 22% | 2% | 15% |
| Méthane | 7% | 12% | 15% | 53% |
| Ethylène | 34% | 44% | 60% | 29% |
| Ethane | 21% | 21% | 22% | 3% |
| C3+ | 2% | 1% | 0% | 0% |

Ce tableau illustre l'augmentation de la teneur en éthylène dans le flux 90 grâce au flux 170 ainsi que son effet stabilisateur. L'augmentation de cette teneur à pression constante engendre l'augmentation de la pression partielle en éthylène, ce qui permet de condenser l'éthylène à plus haut température et avec un coût énergétique moindre.

La puissance consommée par les compresseurs est donnée dans le tableau ci-dessous, dans le cas d'une détente à moyenne pression du courant de recycle intermédiaire 170 : la puissance est exprimée en base 100 par rapport à l'étage de compression 24 du procédé selon WO 2011/051614.

| Puissance consommée (base 100) | Procédé selon WO 2011/051614 | Procédé selon l'invention |
|---|---|---|
| Etage de compression 24 | 100 | 120 |
| Réfrigérant au propylène | 60 | 60 |
| Réfrigérant à l'éthylène | 20 | - |
| Total | 180 | 180 |

Le procédé selon l'invention permet d'obtenir une puissance thermique consommée analogue à celle du procédé avantageux décrit dans WO 2011/051614, mais sans nécessiter la mise en oeuvre d'un cycle à l'éthylène.

Ce résultat est obtenu d'une manière particulièrement surprenante avec une détente du courant de recycle intermédiaire 170 à moyenne pression, et non à basse pression, comme cela est généralement mis en oeuvre pour obtenir des températures après détente les plus froides possibles.

Dans une variante du procédé, représentée en pointillés sur la figure 1, une fraction 180 du courant de gaz combustible 144 de tête est prélevée et est injectée dans le courant gazeux de recyclage 162, après détente dans une vanne de contrôle 182.

Le rapport du débit molaire de la fraction 180 au débit molaire du courant de gaz combustible 144 de tête avant le prélèvement est inférieur à 5% et notamment compris entre 0,5% et 2%.

Dans une autre variante du procédé, représentée en pointillés sur la figure 1, un courant de dérivation 200 est prélevé dans le courant intermédiaire de gaz craqué 114, en amont de l'échangeur thermique intermédiaire 50. Le courant de dérivation 200 est détendu dans une vanne de contrôle 202, puis est injecté dans le courant de recycle intermédiaire détendu 170.

Le rapport du débit molaire du courant de dérivation au débit molaire du courant intermédiaire de gaz craqué 114 avant le prélèvement est inférieur à 5% et notamment compris entre 1% et 3%.

Dans une autre variante du procédé, les liquides 136 et 142 sont introduits séparément dans les échangeurs thermiques 50, 42, 72 pour se réchauffer, avant d'être réintroduits dans le courant de gaz craqué brut 20.

Dans une variante, chaque appareil de détente dynamique 68 comprend une pluralité de turbines de détente dynamique, par exemple de 2 à 3 turbines de détente dynamique. Dans une autre variante, un compresseur additionnel est placé en aval des compresseurs 76A, 76B pour comprimer à une pression plus élevée le gaz combustible 14.

Dans d'autres variantes, l'unité de traitement comprend une pluralité de colonnes de fractionnement comme décrit par exemple dans EP 1 215 459.

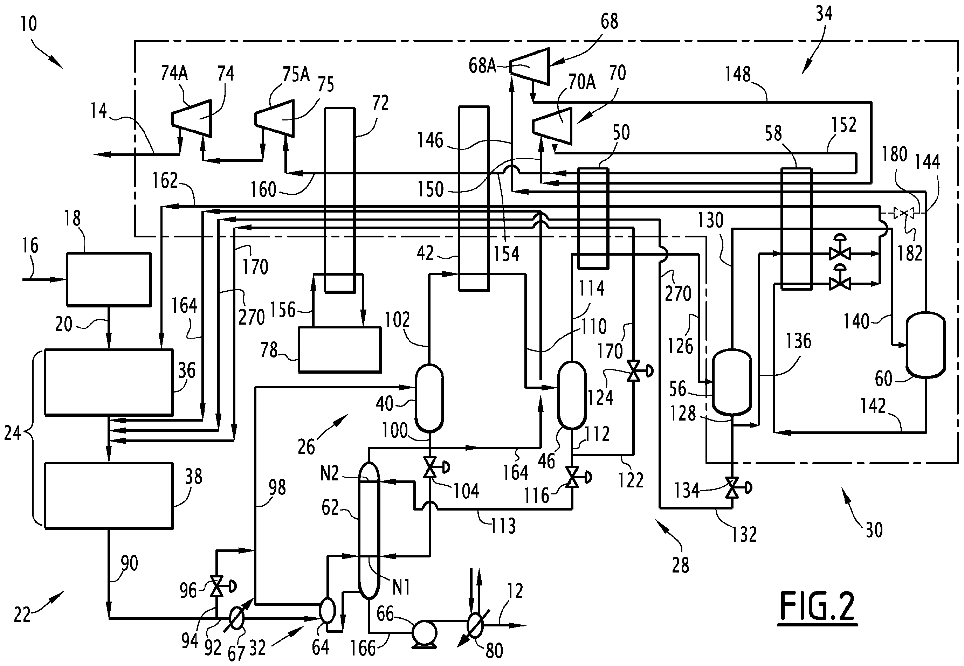

Le procédé illustré partiellement sur la figure 2 diffère de celui illustré sur la figure 1 en ce qu'il comporte la formation d'un premier courant de recycle intermédiaire 170 à partir de la deuxième fraction 122 du liquide amont 112 et la formation d'un deuxième courant de recycle intermédiaire 270, distinct du premier courant de recycle intermédiaire 170, à partir de la première fraction 132 du liquide intermédiaire 128.

Chaque courant 170, 270 est ensuite réchauffé séparément à travers l'échangeur thermique intermédiaire 50, l'échangeur thermique amont 42 et l'échangeur thermique de réchauffage 72.

Chaque courant 170, 270 est ensuite réinjecté dans le gaz craqué brut 20, à l'étage de compression et de refroidissement 24, en amont d'au moins un compresseur 36, 38.

On notera, comme cela est représenté sur la Figure 1, que la totalité du courant de combustible haute pression 144 est réchauffée successivement dans l'échangeur thermique aval, et dans l'échangeur thermique intermédiaire 50 avant d'être introduite en totalité dans le premier appareil de détente dynamique 68.

De même, la totalité du courant de combustible partiellement détendu 148 issu du premier appareil de détente dynamique 68 est passée successivement dans l'échangeur aval 58 et dans l'échangeur intermédiaire 50, avant d'être introduite en totalité dans le deuxième appareil de détente dynamique 70.

La totalité du courant de combustible détendu 152 issu du deuxième appareil de détente dynamique 70 est ensuite introduite dans l'échangeur thermique aval 58 et dans l'échangeur thermique intermédiaire 50.

Ainsi, la récupération de frigories est maximale pour permettre le refroidissement du gaz.

On notera en outre que les ballons 40, 46, 56 et 60 sont de simples ballons séparateurs, et non des colonnes de distillation. Ainsi, ces ballons sont dépourvus de plateaux ou de garnissage

La colonne de fractionnement 62 est une colonne de type « stripper ». Ainsi, le courant de tête 164 riche en méthane issu de la colonne 62 est totalement renvoyé dans le gaz craqué brut 20, sans qu'une fraction de ce courant 164 ne soit condensée pour être envoyée en reflux dans la colonne 62.

## Revendications

1. Procédé de fractionnement d'un courant (20) de gaz craqué issu d'une installation (18) de pyrolyse d'hydrocarbures pour obtenir une coupe (12) riche en éthylène et un courant (14) de combustible pauvre en hydrocarbures en C₂⁺, le procédé comprenant les étapes suivantes :
- compression du courant de gaz craqué brut (20) dans un premier compresseur (36) et dans un deuxième compresseur (38) d'un étage de refroidissement et de compression (24) pour former un courant de gaz craqué comprimé (90) ;
- refroidissement amont et condensation partielle, dans au moins un échangeur thermique amont (42), d'un courant amont (102) de gaz craqué, obtenu à partir du courant de gaz craqué comprimé (90), et séparation d'un liquide amont (112) dans au moins un ballon amont (46) pour former un courant intermédiaire (114) de gaz craqué pré-refroidi à une première température ;
- refroidissement intermédiaire et condensation partielle du courant intermédiaire de gaz craqué (114) dans un échangeur thermique (50) intermédiaire et séparation d'un liquide intermédiaire (128) dans un ballon de séparation intermédiaire (56) pour former un courant aval (130) de gaz craqué refroidi à une deuxième température inférieure à la première température ;
- refroidissement aval et condensation partielle du courant aval de gaz craqué (130) dans au moins un échangeur thermique aval (58) jusqu'à une troisième température inférieure à la deuxième température ;
- introduction du courant aval (140) de gaz craqué partiellement condensé issu de l'échangeur thermique aval (58) dans un séparateur aval (60) ;
- récupération, en tête du séparateur aval (60), d'un courant (144) gazeux de combustible à haute pression, pauvre en hydrocarbures en C₂⁺, et récupération, en pied du séparateur aval, d'un liquide aval (142), riche en hydrocarbures en C₂⁺;
- passage du courant (144) de combustible haute pression à travers l'échangeur aval (58) et l'échangeur intermédiaire (50) pour former un courant (146) de combustible haute pression réchauffé ;
- détente du courant (146) de combustible haute pression réchauffé dans au moins un premier appareil (68) de détente dynamique pour obtenir un courant (148) de combustible partiellement détendu;
- réchauffage du courant (148) de combustible partiellement détendu à travers l'échangeur aval (58) et l'échangeur intermédiaire (50) ;
- traitement d'au moins un courant liquide (113) obtenu lors des étapes de refroidissement amont, de refroidissement intermédiaire et de refroidissement aval pour former la coupe riche en éthylène (12) ;
**caractérisé en ce que** le procédé comprend les étapes suivantes :
- formation d'un courant (170) de recycle intermédiaire détendu à partir d'un liquide (112, 128) obtenu lors des étapes de refroidissement amont, ou/et de refroidissement intermédiaire, en amont de l'étape de refroidissement aval ;
- circulation du courant de recycle intermédiaire (170) au moins dans l'échangeur thermique amont (42) pour refroidir le courant amont de gaz craqué (102) ;
- réintroduction du courant de recycle intermédiaire (170) réchauffé dans le gaz craqué brut (20) entre le premier compresseur (36) et le deuxième compresseur (38) de l'étage de refroidissement et de compression (24),
les étapes de refroidissement amont, intermédiaire et aval s'effectuant sans échange thermique respectivement du courant amont de gaz craqué (102), du courant intermédiaire de gaz craqué (114) et du courant aval de gaz craqué (140) avec un cycle de réfrigération externe, tel qu'un cycle à l'éthylène,
et **en ce que** la pression du courant de recycle intermédiaire détendu (170) est supérieure à 15 % de la pression du courant de gaz craqué comprimé (90).

2. Procédé selon la revendication 1, **caractérisé en ce que** la pression du courant de recycle intermédiaire détendu (170) est comprise entre 20 % et 50 % de la pression du courant de gaz craqué comprimé (90).

3. Procédé selon la revendication 1 ou 2, **caractérisé en ce que** la pression du courant de recycle intermédiaire détendu (170) est supérieure à 5 bars et est notamment comprise entre 5 bars et 20 bars.

4. Procédé selon l'une quelconque des revendications précédentes, **caractérisé en ce que** le débit molaire du courant de recycle intermédiaire détendu (170) est supérieur à 25 % et est notamment compris entre 30 % et 60 % du débit molaire du courant de gaz craqué brut (20).

5. Procédé selon l'une quelconque des revendications précédentes, **caractérisé en ce que** la teneur molaire en éthylène dans le courant de recycle intermédiaire détendu (170) est supérieure à 50 % et est notamment comprise entre 55 % et 65 %.

6. Procédé selon la revendication 5, **caractérisé en ce que** la teneur molaire en éthane dans le courant de recycle intermédiaire (170) est comprise entre 15 % et 30 %, la teneur molaire en méthane dans le courant de recycle intermédiaire (170) étant comprise entre 10 % et 20 %.

7. Procédé selon l'une quelconque des revendications précédentes, **caractérisé en ce que** le rapport de la teneur molaire en éthylène à la teneur molaire en hydrogène dans le courant de gaz craqué brut comprimé (90), après réintroduction du courant de recycle intermédiaire détendu (170) est supérieur à 1,3 fois le rapport de la teneur molaire en éthylène à la teneur molaire en hydrogène dans le courant de gaz craqué brut (20), avant la réintroduction du courant de recycle intermédiaire détendu (170) dans le gaz craqué brut (20).

8. Procédé selon l'une quelconque des revendications précédentes, **caractérisé en ce que** la température du courant de recycle intermédiaire (170) est comprise entre -75 °C et -95 °C, après détente, et avant introduction dans un échangeur thermique.

9. Procédé selon l'une quelconque des revendications précédentes, **caractérisé en ce qu'**il comporte la formation d'un courant de recyclage détendu (162) à partir d'au moins une fraction d'un liquide intermédiaire (128) et/ou d'au moins une fraction du liquide aval (142), le courant de recyclage détendu (162) étant introduit dans l'échangeur thermique aval (58), et/ou dans l'échangeur thermique intermédiaire (50), avant d'être mélangé au courant de gaz craqué brut (20) avant le passage du courant de gaz craqué brut (20) dans au moins un compresseur (38) de l'étage de refroidissement et de compression (24), la pression du courant de recyclage détendu (162) étant inférieure à la pression du courant de recycle intermédiaire détendu (170).

10. Procédé selon la revendication 9, **caractérisé en ce qu'**il comporte l'injection d'au moins une fraction (180) prélevée dans le courant gazeux de combustible haute pression (144) dans le courant de recyclage détendu (162).

11. Procédé selon l'une quelconque des revendications précédentes, **caractérisé en ce qu'**il comporte le prélèvement d'un courant de dérivation (200) dans le courant intermédiaire de gaz craqué (114), en amont de l'échangeur thermique intermédiaire (50) et l'injection du courant de dérivation (200), après détente, dans le courant de recycle intermédiaire détendu (170).

12. Procédé selon l'une quelconque des revendications précédentes, **caractérisé en ce qu'**il comporte la formation d'au moins un courant de recycle intermédiaire (170) à partir du liquide amont (112) issu du ballon de séparation amont (46) et la formation d'au moins un courant de recycle intermédiaire (170 ; 270) à partir du liquide intermédiaire (128) issu du ballon de séparation intermédiaire (56).

13. Procédé selon l'une quelconque des revendications précédentes, **caractérisé en ce qu'**il comporte la mise en relation d'échange thermique d'au moins une fraction (92) du courant de gaz craqué comprimé (90) avec un fluide réfrigérant circulant dans un cycle de réfrigération externe, puis son introduction dans un ballon séparateur amont (40) pour former le courant amont de gaz craqué (102).

14. Installation (22) de fractionnement d'un courant (20) de gaz craqué issu d'une installation (18) de pyrolyse d'hydrocarbures pour obtenir une coupe (12) riche en éthylène et un courant (14) de combustible pauvre en hydrocarbures en C₂⁺, l'installation de fractionnement (22) comprenant :
- un étage (24) de refroidissement et de compression du courant de gaz craqué brut (20) comprenant un premier compresseur (36) et un deuxième compresseur (38), pour former un courant de gaz craqué comprimé (90) ;
- un ensemble amont (26) de refroidissement et de condensation partielle d'un courant amont (102) de gaz craqué, obtenu à partir du courant de gaz craqué comprimé (90), l'ensemble amont (26) comprenant au moins un échangeur thermique amont (42), et au moins un ballon amont (46) de séparation d'un liquide amont (112) pour former un courant intermédiaire (114) de gaz craqué pré-refroidi à une première température ;
- un ensemble intermédiaire (28) de refroidissement et de condensation partielle du courant intermédiaire de gaz craqué (114), l'ensemble intermédiaire (28) comprenant un échangeur thermique (50) intermédiaire et un ballon de séparation intermédiaire (56) d'un liquide intermédiaire (128) pour former un courant aval (130) de gaz craqué refroidi à une deuxième température inférieure à la première température ;
- un ensemble aval (30) de refroidissement et de condensation partielle du courant aval de gaz craqué (130) pour refroidir le courant aval de gaz craqué (130) jusqu'à une troisième température inférieure à la deuxième température, l'ensemble aval (30) comprenant au moins un échangeur thermique aval (58) ;
- un séparateur aval (60) et un ensemble d'introduction du courant aval (140) de gaz craqué partiellement condensé issu de l'échangeur thermique aval (58) dans le séparateur aval (60) ;
- un ensemble de récupération, en tête du séparateur aval (60), d'un courant (144) gazeux de combustible à haute pression, pauvre en hydrocarbures en C₂⁺, et de récupération, en pied du séparateur aval (60), d'un liquide aval (142), riche en hydrocarbures en C₂⁺;
- un ensemble de passage du courant (144) de combustible haute pression à travers l'échangeur aval (58) et l'échangeur intermédiaire (50) pour former un courant (146) de combustible haute pression réchauffé ;
- au moins un premier appareil (68) de détente dynamique du courant (146) de combustible haute pression réchauffé pour obtenir un courant (148) de combustible partiellement détendu ;
- un ensemble de passage du courant (148) de combustible partiellement détendu à travers l'échangeur aval (58) et l'échangeur intermédiaire (50) pour réchauffer le courant de combustible partiellement détendu (148) ;
- un ensemble (32) de traitement d'au moins un courant liquide (113) obtenu dans au moins un des ensembles (26, 28, 30) de refroidissement amont, de refroidissement intermédiaire, et de refroidissement aval pour former la coupe riche en éthylène (12) ;
**caractérisée en ce que** l'installation (22) comprend:
- un ensemble de formation d'un courant (170) de recycle intermédiaire détendu à partir d'un liquide (112, 128) obtenu dans au moins un des ensembles (26, 28) de refroidissement amont, ou/et de refroidissement intermédiaire, en amont de l'ensemble (30) de refroidissement aval ;
- un ensemble de mise en circulation du courant de recycle intermédiaire (170) au moins dans l'échangeur thermique amont (42) pour refroidir le courant amont de gaz craqué (102) ;
- un ensemble de réintroduction du courant de recycle intermédiaire (170) réchauffé dans le gaz craqué brut (20) entre un premier compresseur (36) et un deuxième compresseur (38) de l'étage de refroidissement et de compression (24), la pression du courant de recycle intermédiaire détendu (170) étant supérieure à 15 % et avantageusement comprise entre 20 % et 50 % de la pression du courant de gaz craqué comprimé (90),
les ensembles de refroidissement amont, intermédiaire et aval étant configurés pour refroidir respectivement le courant amont de gaz craqué (102), le courant intermédiaire de gaz craqué (114) et le courant aval de gaz craqué (140) sans échange thermique avec un cycle de réfrigération externe, tel qu'un cycle à l'éthylène.

## Patentansprüche

1. Verfahren zur Fraktionierung eines Spaltgas-Stroms (20), der von einer Installation (18) zur Pyrolyse von Kohlenwasserstoffen stammt, zum Erlangen einer Fraktion (12) reich an Ethylen und eines Brennstoff-Stroms (14) arm an Kohlenwasserstoffen von C₂⁺, wobei das Verfahren die folgenden Schritte aufweist:
- Verdichten des Roh-Spaltgas-Stroms (20) in einem ersten Verdichter (36) und in einem zweiten Verdichter (38) einer Kühlungs- und Verdichtungs-Stufe (24) zum Bilden eines verdichteten Spaltgas-Stroms (90),
- Stromaufwärts-Kühlen und Teil-Kondensieren, in wenigstens einem Stromaufwärts-Wärmetauscher (42), eines Stromaufwärts-Spaltgas-Stroms (102), der erlangt wird ausgehend von dem verdichteten Spaltgas-Strom (90), und Separieren einer Stromaufwärts-Flüssigkeit (112) in wenigstens einen Stromaufwärts-Behälter (46) zum Bilden eines Zwischen-Spaltgas-Stroms (114), der auf eine erste Temperatur vorgekühlt ist,
- Zwischen-Kühlen und Teilkondensieren des Zwischen-Spaltgas-Stroms (114) in einem Zwischen-Wärmetauscher (50) und Separieren einer Zwischen-Flüssigkeit (128) in einen Zwischen-Separations-Behälter (56) zum Bilden eines Stromabwärts-Spaltgas-Stroms (130), der auf eine zweite Temperatur gekühlt ist, die kleiner als die erste Temperatur ist,
- Stromabwärts-Kühlen und Teilkondensieren des Stromabwärts-Spaltgas-Stroms (130) in wenigstens einem Stromabwärts-Wärmetauscher (58) bis zu einer dritten Temperatur, die kleiner als die zweite Temperatur ist,
- Einbringen des teilkondensierten Stromabwärts-Spaltgas-Stroms (140), der von dem Stromabwärts-Wärmetauscher (58) stammt, in einen Stromabwärts-Separator (60),
- Rückgewinnen, am Kopf eines Stromabwärts-Separators (60), eines Brennstoff-Gas-Stroms (144) hohen Drucks, der arm an Kohlenwasserstoffen von C₂⁺ ist, und Rückgewinnen, am Fuß des Stromabwärts-Separators (60), einer Stromabwärts-Flüssigkeit (142), die reich an Kohlenwasserstoffen von C₂⁺ ist,
- Passieren des Brennstoff-Stroms (144) hohen Drucks durch den Stromabwärts-Tauscher (58) und den Zwischen-Tauscher (50) zum Bilden eines erwärmten Brennstoff-Stroms (146) hohen Drucks,
- Expandieren des erwärmten Brennstoff-Stroms (146) hohen Drucks in wenigstens einer ersten Apparatur (68) zur dynamischen Expansion zum Erlangen eines teilexpandierten Brennstoff-Stroms (148),
- Erwärmen des teilexpandierten Brennstoff-Stroms (148) durch den Stromabwärts-Tauscher (58) und den Zwischen-Tauscher (50),
- Behandeln wenigstens eines Flüssigkeits-Stroms (113), der während der Schritte des Stromaufwärts-Kühlens, des ZwischenKühlens und des Stromabwärts-Kühlens erlangt wurde, zum Bilden der Ethylen-reichen Fraktion (12),
**dadurch gekennzeichnet, dass** das Verfahren die folgenden Schritte aufweist:
- Bilden eines expandierten Zwischen-Recycle-Stroms (170) ausgehend von einer Flüssigkeit (112, 128), die während des Schritts des Stromaufwärts-Kühlens und/oder des Schritts des Zwischenkühlens erlangt wurde, stromaufwärts des Schritts des Stromabwärts-Kühlens,
- Zirkulieren des Zwischen-Recycle-Stroms (170) zumindest in dem Stromaufwärts-Wärmetauscher (42) zum Kühlen des Stromaufwärts-Spaltgas-Stroms (102),
- Wiedereinbringen des erwärmten Zwischen-Recycle-Stroms (170) in das Roh-Spaltgas (20) zwischen dem ersten Verdichter (36) und dem zweiten Verdichter (38) der Kühlungs- und Verdichtungs-Stufe (24),
wobei die Schritte des Stromaufwärts-, Zwischen- und Stromabwärts-Kühlens erfolgen ohne Wärmetausch respektive des Stromaufwärts-Spaltgas-Stroms (102), des Zwischen-Spaltgas-Stroms (114) und des Stromabwärts-Spaltgas-Stroms (140) mit einem externen Kältekreis, wie z.B. einem Ethylen-Kreis, und dass der Druck des expandierten Zwischen-Recycle-Stroms (170) größer als 15% des Drucks des verdichteten Spaltgas-Stroms (90) ist.

2. Verfahren gemäß Anspruch 1, **dadurch gekennzeichnet, dass** der Druck des expandierten Zwischen-Recycle-Stroms (170) zwischen 20% und 50% des Drucks des verdichteten Spaltgas-Stroms (90) liegt.

3. Verfahren gemäß Anspruch 1 oder 2, **dadurch gekennzeichnet, dass** der Druck des expandierten Zwischen-Recycle-Stroms (170) größer als 5 Bar ist und insbesondere zwischen 5 Bar und 20 Bar liegt.

4. Verfahren gemäß irgendeinem der vorhergehenden Ansprüche, **dadurch gekennzeichnet, dass** der molare Durchfluss des expandierten Zwischen-Recycle-Stroms (170) größer als 25% und insbesondere zwischen 30% und 60% des molaren Durchflusses des Roh-Spaltgas-Stroms (20) ist.

5. Verfahren gemäß irgendeinem der vorhergehenden Ansprüche, **dadurch gekennzeichnet, dass** der Molgehalt an Ethylen in dem expandierten Zwischen-Recycle-Strom (170) größer als 50% und insbesondere zwischen 55% und 65% ist.

6. Verfahren gemäß Anspruch 5, **dadurch gekennzeichnet, dass** der Molgehalt an Ethan in dem Zwischen-Recycle-Strom (170) zwischen 15% und 30% ist, wobei der Molgehalt an Methan in dem Zwischen-Recycle-Strom (170) zwischen 10% und 20% ist.

7. Verfahren gemäß irgendeinem der vorhergehenden Ansprüche, **dadurch gekennzeichnet, dass** das Verhältnis des Molgehalts an Ethylen zu dem Molgehalt an Wasserstoff in dem verdichteten Roh-Spaltgas-Strom (90) nach dem Wiedereinbringen des expandierten Zwischen-Recycle-Stroms (170) größer ist als das 1,3-fache des Verhältnisses des Molgehalts an Ethylen zu dem Molgehalt an Wasserstoff in dem Roh-Spaltgas-Strom (20) vor dem Wiedereinbringen des expandierten Zwischen-Recycle-Stroms (170) in das Roh-Spaltgas (20).

8. Verfahren gemäß irgendeinem der vorhergehenden Ansprüche, **dadurch gekennzeichnet, dass** die Temperatur des Zwischen-Recycle-Stroms (170) nach der Expansion und vor der Einbringung in einen Wärmetauscher zwischen -75 °C und -95 °C liegt.

9. Verfahren gemäß irgendeinem der vorhergehenden Ansprüche, **dadurch gekennzeichnet, dass** es aufweist die Bildung eines expandierten Recycling-Stroms (162) ausgehend von wenigstens einer Fraktion einer Zwischen-Flüssigkeit (128) und/oder wenigstens einer Fraktion der Stromabwärts-Flüssigkeit (142), wobei der expandierte Recycling-Strom (162) in den Stromabwärts-Wärmetauscher (58) und/oder in den Zwischen-Wärmetauscher (50) eingegeben wird, bevor er mit dem Roh-Spaltgas-Strom (20) gemischt wird vor dem Passieren des Roh-Spaltgas-Stroms (20) durch wenigstens einen Verdichter (38) der Kühlungs- und Verdichtungs-Stufe (24), wobei der Druck des expandierten Recycling-Stroms (162) kleiner ist als der Druck des expandierten Zwischen-Recycle-Stroms (170).

10. Verfahren gemäß Anspruch 9, **dadurch gekennzeichnet, dass** es aufweist das Einspeisen wenigstens einer Fraktion (180), die abgezweigt ist aus dem Brennstoff-Gas-Strom hohen Drucks (144), in den expandierten Recycling-Strom (162).

11. Verfahren gemäß irgendeinem der vorhergehenden Ansprüche, **dadurch gekennzeichnet, dass** es aufweist Abzweigen eines Umleitungs-Stroms (200) aus dem Zwischen-Spaltgas-Strom (114) stromaufwärts des Zwischen-Wärmetauschers (50) und Einspeisen des Umleitungs-Stroms (200), nach dem Expandieren, in den expandierten Zwischen-Recycle-Strom (170).

12. Verfahren gemäß irgendeinem der vorhergehenden Ansprüche, **dadurch gekennzeichnet, dass** es aufweist das Bilden wenigstens eines Zwischen-Recycle-Stroms (170) ausgehend von der Stromaufwärts-Flüssigkeit (112), die von dem Stromaufwärts-Separations-Behälter (46) stammt, und das Bilden wenigstens eines Zwischen-Recycle-Stroms (170; 270) ausgehend von der Zwischen-Flüssigkeit (128), die von dem Zwischen-Separations-Behälter (56) stammt.

13. Verfahren gemäß irgendeinem der vorhergehenden Ansprüche, **dadurch gekennzeichnet, dass** es aufweist das Bewirken von Wärmetausch von wenigstens einer Fraktion (92) des verdichteten Spaltgas-Stroms (90) mit einem Kältefluid, das in einem externen Kälte-Kreis zirkuliert, und dann deren Einbringung in einen Stromaufwärts-Separations-Behälter (40) zum Bilden des Stromaufwärts-Spaltgas-Stroms (102).

14. Installation (22) zur Fraktionierung eines Spaltgas-Stroms (20), der von einer Installation (18) zur Pyrolyse von Kohlenwasserstoffen stammt, zum Erlangen einer Fraktion (12) reich an Ethylen und eines Brennstoff-Stroms (14) arm an Kohlenwasserstoffen von C₂⁺, wobei die Installation zur Fraktionierung (22) aufweist:
- eine Stufe (24) zur Kühlung und Verdichtung des Roh-Spaltgas-Stroms (20), aufweisend einen ersten Verdichter (36) und einen zweiten Verdichter (38), zum Bilden eines verdichteten Spaltgas-Stroms (90),
- eine Stromaufwärts-Einrichtung (26) zur Kühlung und Teilkondensierung eines Stromaufwärts-Spaltgas-Stroms (102), der ausgehend von dem verdichteten Spaltgas-Strom (90) erlangt ist, wobei die Stromaufwärts-Einrichtung (26) aufweist wenigstens einen Stromaufwärts-Wärmetauscher (42) und wenigstens einen Stromaufwärts-Behälter (46) zur Separation einer Stromaufwärts-Flüssigkeit (112) zum Bilden eines Zwischen-Spaltgas-Stroms (114), der auf eine erste Temperatur vorgekühlt ist,
- eine Zwischen-Einrichtung (28) zur Kühlung und zur Teilkondensierung des Zwischen-Spaltgas-Stroms (114), wobei die Zwischen-Einrichtung (28) aufweist einen Zwischen-Wärmetauscher (50) und einen Behälter zur Zwischen-Separation (56) einer Zwischen-Flüssigkeit (128) zum Bilden eines Stromabwärts-Spaltgas-Stroms (130), der auf eine zweite Temperatur gekühlt ist, die kleiner als die erste Temperatur ist,
- Stromabwärts-Einrichtung (30) zur Kühlung und zur Teilkondensierung des Stromabwärts-Spaltgas-Stroms (130) zum Kühlen des Stromabwärts-Spaltgas-Stroms (130) bis auf eine dritte Temperatur, die kleiner als die zweite Temperatur ist, wobei die Stromabwärts-Einrichtung (30) aufweist wenigstens einen Stromabwärts-Wärmetauscher (58),
- einen Stromabwärts-Separator (60) und eine Einrichtung zur Einbringung des teilkondensierten Stromabwärts-Spaltgas-Stroms (140), der von dem Stromabwärts-Wärmetauscher (58) stammt, in den Stromabwärts-Separator (60),
- eine Einrichtung zur Wiedergewinnung, am Kopf des Stromaufwärts-Separators (60), eines Brennstoff-Gas-Stroms (144) hohen Drucks, der arm an Kohlenwasserstoffen von C₂⁺ ist, und zur Wiedergewinnung, am Fuß des Stromabwärts-Separators (60), einer Stromabwärtsflüssigkeit (142), die reich an Kohlenwasserstoffen von C₂⁺ ist,
- eine Einrichtung zum Passieren des Brennstoff-Stroms (144) hohen Drucks durch den Stromabwärts-Tauscher (58) und den Zwischen-Tauscher (50) zum Bilden eines erwärmten Brennstoff-Stroms (146) hohen Drucks,
- wenigstens eine erste Apparatur (68) zur dynamischen Expansion des erwärmten Brennstoff-Stroms (146) hohen Drucks zum Erlangen eines teilexpandierten Brennstoff-Stroms (148),
- eine Einrichtung zum Passieren des teilexpandierten Brennstoff-Stroms (148) durch den Stromabwärts-Tauscher (58) und den Zwischen-Tauscher (50) zum Erwärmen des teilexpandierten Brennstoff-Stroms (148),
- eine Einrichtung (32) zur Behandlung wenigstens eines Flüssigkeits-Stroms (113), der erlangt ist in wenigstens einer der Einrichtungen (26, 28, 30) zur Stromaufwärts-Kühlung, zur Zwischen-Kühlung und zur Stromabwärts-Kühlung, zum Bilden der Ethylen-reichen Fraktion (12),
**dadurch gekennzeichnet, dass** die Installation (22) aufweist:
- eine Einrichtung zur Bildung eines expandierten Zwischen-Recycle-Stroms (170) ausgehend von einer Flüssigkeit (112, 128), die erlangt ist in wenigstens einer von der Einrichtung zur Stromaufwärts-Kühlung und/oder der Einrichtung zur Zwischen-Kühlung (26, 28), stromaufwärts der Einrichtung (30) zur Stromabwärts-Kühlung,
- eine Einrichtung zum Zirkulieren des Zwischen-Recycle-Stroms (170) wenigstens in dem Stromaufwärts-Wärmetauscher (42) zum Kühlen des Stromaufwärts-Spaltgas-Stroms (102),
- eine Einrichtung zur Wiedereinbringung des erwärmten Zwischen-Recycle-Stroms (170) in das Roh-Spaltgas (20) zwischen einem ersten Verdichter (36) und einem zweiten Verdichter (38) der Kühlungs- und Verdichtungs-Stufe (24), wobei der Druck des expandierten Zwischen-Recycle-Stroms (170) größer als 15% und vorteilhafterweise zwischen 20% und 50% des Drucks des verdichteten Spaltgas-Stroms (90) ist,
wobei die Einrichtungen zur Stromaufwärts-, Zwischen- und Stromabwärts-Kühlung konfiguriert sind zum Kühlen respektive des Stromaufwärts-Spaltgas-Stroms (102), des Zwischen-Spaltgas-Stroms (114) und des Stromabwärts-Spaltgas-Stroms (140) ohne Wärmetausch mit einem externen Kälte-Kreis, so wie z.B. einem Ethylen-Kreis.

## Claims

1. A method for fractionating a stream (20) of cracked gas from a hydrocarbon pyrolysis plant (18) to obtain an ethylene-rich cut (12) and a C₂⁺ hydrocarbon-lean fuel stream (14), the method comprising the following steps:
- compression of the stream of raw cracked gas (20) in a first compressor (36), and in a second compressor (38) of a cooling and compression stage (24) to form a compressed cracked gas stream (90);
- upstream cooling and partial condensation, in at least one upstream heat exchanger (42), of an upstream stream (102) of cracked gas, obtained from the compressed cracked gas stream (90), and separation of an upstream liquid (112) in at least one upstream balloon (46) to form an intermediate stream (114) of cracked gas pre-cooled at first temperature;
- intermediate cooling and partial condensation of the intermediate stream of cracked gas (114) in an intermediate heat exchanger (50) and separation of an intermediate liquid (128) in an intermediate separating balloon (56) to form a downstream stream (130) of cracked gas cooled to a second temperature lower than the first temperature;
- downstream cooling and partial condensation of the downstream stream of cracked gas (130) in at least one downstream heat exchanger (58) to a third temperature lower than the second temperature;
- introduction of the downstream stream (140) of partially condensed cracked gas from the downstream heat exchanger (58) in a downstream separator (60);
- recovery, at the head of the downstream separator (60), of a gas stream (144) of high-pressure fuel, lean in C₂⁺ hydrocarbons, and recovery, at the bottom of the downstream separator, of a downstream liquid (142), rich in C₂⁺ hydrocarbons;
- passage of the stream (144) of high-pressure fuel through the downstream exchanger (58) and the intermediate exchanger (50) to form a heated high-pressure fuel stream (146);
- expansion of the heated high-pressure fuel stream (146) in at least one first dynamic expansion device (68) to obtain a stream (148) of partially expanded fuel;
- heating of the stream (148) of partially expanded fuel through the downstream exchanger (58) and the intermediate exchanger (50);
- treatment of at least one liquid stream (113) obtained during the upstream cooling, intermediate cooling and downstream cooling steps to form the ethylene-rich cut (12);
**characterized in that** the method comprises the following steps:
- forming an expanded intermediate recirculation stream (170) from a liquid (112, 128) obtained during the upstream cooling and/or intermediate cooling steps, upstream from the downstream cooling step;
- circulating the intermediate recirculation stream (170) at least in the upstream heat exchanger (42) to cool the upstream stream of cracked gas (102);
- reintroducing the reheated intermediate recirculation stream (170) in the raw cracked gas (20) between the first compressor (36) and the second compressor (38) of the cooling and compression stage (24),
the upstream, intermediate and downstream cooling steps being carried out without heat exchange respectively of the upstream stream of cracked gas (102), the intermediate stream of cracked gas (114) and the downstream stream of cracked gas (140) with an external refrigeration cycle, such as an ethylene cycle, and **in that** the pressure of the expanded intermediate recirculation stream (170) is greater than 15% of the pressure of the compressed cracked gas stream (90).

2. The method according to claim 1, **characterized in that** the pressure of the expanded intermediate recirculation stream (170) is comprised between 20% and 50% of the pressure of the compressed cracked gas stream (90).

3. The method according to claim 1 or 2, **characterized in that** the pressure of the expanded intermediate recirculation stream (170) is greater than 5 bars and is in particular comprised between 5 bars and 20 bars.

4. The method according to any one of the preceding claims, **characterized in that** the molar flow rate of the expanded intermediate recirculation stream (170) is greater than 25% and is in particular comprised between 30% and 60% of the molar flow rate of the stream of raw cracked gas (20).

5. The method according to any one of the preceding claims, **characterized in that** the molar ethylene content in the expanded intermediate recirculation stream (170) is greater than 50%, and is in particular comprised between 55% and 65%.

6. The method according to claim 5, **characterized in that** the molar content level of ethane in the intermediate recirculation stream (170) is comprised between 15% and 30%, the molar content level of methane in the intermediate recirculation stream (170) being comprised between 10% and 20%.

7. The method according to any one of the preceding claims, **characterized in that** the ratio of the molar ethylene content to the molar hydrogen content in the compressed raw cracked gas stream (90), after reintroduction of the expanded intermediate recirculation stream (170), is greater than 1.3 times the ratio of the molar ethylene content to the molar hydrogen content in the raw cracked gas stream (20), before the reintroduction of the expanded intermediate recirculation stream (170) in the raw cracked gas stream (20).

8. The method according to any one of the preceding claims, **characterized in that** the temperature of the intermediate recirculation stream (170) is comprised between - 75°C and -95°C, after expansion, and before introduction in a heat exchanger.

9. The method according to any one of the preceding claims, **characterized in that** it comprises the formation of an expanded recirculation stream (162) from at least one fraction of an intermediate liquid (128) and/or at least one fraction of the downstream liquid (142), the expanded recirculation stream (162) being introduced into the downstream heat exchanger (58), and/or into the intermediate heat exchanger (50), before being mixed with the raw cracked gas stream before the passage of the raw cracked gas stream (20) in at least one compressor (38) of the cooling and compression stage (24), the pressure of the expanded recirculation stream (162) being lower than the pressure of the expanded intermediate recirculation stream (170).

10. The method according to claim 9, **characterized in that** it comprises the injection of at least one fraction (180) taken from the high-pressure fuel gas stream (144) into the expanded recirculation stream (162).

11. The method according to any one of the preceding claims, **characterized in that** it comprises the withdrawal of a bypass stream (200) from the intermediate cracked gas stream (114), upstream from the intermediate heat exchanger (50) and the injection of the bypass stream (200), after expansion, in the expanded intermediate recirculation stream (170).

12. The method according to any one of the preceding claims, **characterized in that** it comprises forming at least one intermediate recirculation stream (170) from the upstream liquid (112) coming from the upstream separator balloon (46) and the formation of at least one intermediate recirculation stream (170; 270) from the intermediate liquid (128) from the intermediate separator balloon (56).

13. The method according to any one of the preceding claims, **characterized in that** it comprises providing a heat exchange relationship between at least one fraction (92) of the compressed cracked gas stream (90) and a refrigerant circulating in an outside refrigeration cycle, then its introduction into an upstream separator balloon (40) to form the upstream stream of cracked gas (102).

14. A fractionating plant (22) for a first stream of cracked gas (20) coming from a hydrocarbon pyrolysis plant (18) to obtain an ethylene-rich cut (12) and a C₂⁺ hydrocarbon-lean fuel stream (14), the fractionating plant (22) comprising:
- a cooling and compression stage (24) for the stream of raw cracked gas (20) comprising a first compressor (36), a second compressor (38), to form a stream of compressed cracked gas (90);
- an upstream cooling and partial condensation assembly (26) for an upstream stream of cracked gas (102), obtained from the stream of compressed cracked gas (90), the upstream assembly (26) comprising at least one upstream heat exchanger (42), and at least one upstream separator balloon (46) for an upstream liquid (112) to form an intermediate stream (114) of cracked gas pre-cooled to a first temperature;
- an intermediate cooling and partial condensation assembly (28) for the intermediate stream of cracked gas (114), the intermediate assembly (28) comprising an intermediate heat exchanger (50) and an intermediate separating balloon (56) for an intermediate liquid (128) to form a downstream stream (130) of cracked gas cooled to a second temperature below the first temperature;
- a downstream cooling and partial condensation assembly (30) for the downstream stream of cracked gas (130) to cool the downstream stream of cracked gas (130) to a third temperature below the second temperature, the downstream assembly (30) comprising at least one downstream heat exchanger (58);
- a downstream separator (60) and an assembly for introducing the downstream stream (140) of partially condensed cracked gas coming from the downstream heat exchanger (58) into the downstream separator (60);
- a recovery assembly, at the head of the downstream separator (60), for recovering a gaseous stream (144) of high-pressure fuel, lean in C₂⁺ hydrocarbons, and for recovering, at the bottom of the downstream separator (60), a downstream liquid (142), rich in C₂⁺ hydrocarbons;
- a passage assembly (144) for the stream of high-pressure fuel through the downstream exchanger (58) and the intermediate exchanger (50) to form a stream (146) of reheated high-pressure fuel;
- at least first one dynamic expansion device (68) for the stream (146) of reheated high-pressure fuel to obtain a partially expanded fuel stream (148);
- a passage assembly for the stream (148) of partially expanded fuel through the downstream exchanger (58) and the intermediate exchanger (50) to reheat the stream of partially expanded fuel (148);
- a treatment assembly (32) for at least one liquid stream (113) obtained in at least one of the upstream cooling, intermediate cooling, and downstream cooling assemblies (26, 28, 30) to form the ethylene-rich cut (12);
**characterized in that** the plant (22) comprises:
- an assembly for forming an expanded intermediate recirculation stream (170) from a liquid (112, 128) obtained in at least one of the upstream cooling and/or intermediate cooling assemblies (26, 28), upstream from the downstream cooling assembly (30);
- an assembly for circulating the intermediate recirculation stream (170) at least in the upstream heat exchanger (42) to cool the upstream stream of cracked gas (102);
- an assembly for reintroducing the heated intermediate recirculation stream (170) into the raw cracked gas (20) between a first compressor (36) and a second compressor (38) of the cooling and compression stage (24), the pressure of the expanded intermediate recirculation stream (170) being greater than 15%, and advantageously comprised between 20% and 50%, of the pressure of the compressed cracked gas stream (90),
the upstream, intermediate and downstream cooling assemblies being configured to respectively cool the upstream stream of cracked gas (102), the intermediate stream of cracked gas (114) and the downstream stream of cracked gas (140) without heat exchange with an outside refrigeration cycle, such as an ethylene cycle.
